# EUROPEAN PATENT APPLICATION

(11) **EP 3 679 866 A1**
(43) Date of publication of application: **15.07.2020**
(21) Application number: 20160920.3
(22) Date of filing: 17.03.2017
(51) Int. Cl.: A61B 17/02, A61B 17/32, A61B 17/3209, A61B 17/00, A61B 90/00

(54) **SURGICAL VISUAL FIELD ENHANCER AND SURGICAL KNIFE**

(62) Divisional of application: 17161543.8
(71) Applicant: The Prince Of Songkla University, Songkla 90110 (TH)
(72) Inventor: Wongsiri, Sunton, 90110 Songkla (TH); Patcharakitti, Atcharee, 11140 Nonthaburi (TH)
(74) Representative: Hoffmann Eitle

(57) **Abstract**

The present invention relates to a surgical knife comprisng: a tool body for gripping the tool having a longitudinal axis; a separating end; and a cutting end, wherein the separating end has a blunt tip for separating nerves from ligaments, and wherein the cutting end comprises: a blade for cutting a ligament; a first visual marker proximate the blade.

## Description

### Technical Field

The present invention relates to a surgical tool for use in surgery. In particular the invention relates to a tool for use in surgery for the alleviation of carpal tunnel syndrome.

### Background

Carpal Tunnel syndrome is caused by compression of the median nerve. One way to reduce the compression of the median nerve is by carpal tunnel release surgery. This involves cutting of the transverse carpal ligament. During such surgery, it is desirable to make an incision of the minimum size in order to aid healing and reduce the chance of infection. One problem with this minimally invasive approach is that the visual field for the surgeon is reduced, leading to a higher risk that a nerve might be cut.

US 2013/0211201 A1 discloses a visual field enhancer, which can be used to create a tunnel under the skin in order to improve the visual field of the surgeon. Other instruments may then be passed through said tunnel in order to carry out the surgery.

Within this technical field, however, further developments are required in order to reduce the required incision size and to further reduce the risk that a nerve might be cut during the procedure.

### Summary of the Invention

Accordingly, it is an object of the present invention to provide a surgical tool for providing a sufficient visual field to a surgeon when in use, while reducing the size of incision required for the insertion of said tool.

According to the invention, there is provided a surgical tool comprising: a tool body having a longitudinal axis and being for gripping the tool; the tool body comprising a first end, and a vision enhancer extending from the tool body at the first end, the vision enhancer comprising: a tunnel base, and a first tunnel wall extending from the tunnel base at an angle of between 90 and 120 degrees to the tunnel base, wherein the tunnel base and first tunnel wall form a concave section for the forming of a tunnel through soft tissue.

The term vision enhancer is used herein to mean any tool or part of a tool which can improve the visual field of a surgeon when correctly deployed. In the present invention, this is achieved by formation of a space within the tissue.

While the tunnel is defined as comprising a tunnel base and first tunnel wall, it is realised that the tunnel may be formed of a single piece of material which does not have clearly delimited sections. The tunnel would, however, still be considered as having a tunnel base and tunnel wall.

The term tunnel base is used herein to mean a section of a tunnel which joins to the tool body and can be the part from which other sections of the tunnel extend.

The term tunnel wall is used herein to mean an outer portion of the tunnel which goes to an extremity of the tunnel.

In an embodiment, a groove may extend along a line joining the first tunnel wall and the tunnel base.

With such a configuration, the tunnel wall may hinge along said line relative to the tunnel base in order to fit through a smaller incision, while maintaining a good tunnel size under the skin.

In an embodiment, the vision enhancer may comprise a second tunnel wall.

With such a configuration, the vision enhancer may form a larger tunnel, giving a wider field of view or vision to the surgeon.

In an embodiment, the vision enhancer may comprise at least two grooves, each groove extending along a line between a tunnel wall and the tunnel base.

With such a configuration, both tunnel walls may hinge in order to increase the reduction in size of the vision enhancer in order to fit through an even smaller incision while maintaining a good tunnel size under the skin.

The first and the second tunnel walls are preferably spaced apart from each other, in a direction perpendicular to the longitudinal axis, by the dimension of the tunnel base in that direction. In this configuration, the outer sides of the first and second tunnel walls can be contiguous and continuous with the outside of the tunnel base, which assists insertion at lower risk of injury to the patient.

In an embodiment, the vision enhancer may be flexible such that the distance between the ends of the tunnel walls opposite the tunnel base can be reduced by bending of the material, optionally wherein the distance can be reduced by between about 10% and about 20%, for example from 18 mm to 15 mm. With such a configuration, the vision enhancer may occupy a smaller space when being inserted via an incision while maintaining a good tunnel size under the skin, with the optional dimensions, approximate or exact, having proven to be preferred in view of matching the average anatomy of the carpal tunnel syndrome patient population, deviations from these values still fall within the ambit of the present invention.

In an embodiment, the vision enhancer protrudes from the tool body at an angle of between 80 and 85 degrees from to the longitudinal axis of the tool body, preferably at an angle of about 82 degrees.

With such a configuration, the tool body may be positioned such as to avoid impeding the surgeon while the vision enhancer is in use.

In an embodiment, the tunnel wall(s) may extend from the tunnel base at an angle between 90 degrees and 120 degrees, preferably between 90 degrees and 95 degrees.

With such a configuration, the vision enhancer provides a sufficiently large tunnel without the tunnel base or walls being unsatisfactorily large.

In an embodiment, the vision enhancer may comprise ridges on the outside of the vision enhancer.

With such a configuration, in which the tunnel is considered to be the inside, the vision enhancer will have a stronger frictional interaction with tissue adjacent the outside and so reduce slippage of the tool. This gives a more stable base for the surgeon to work with.

In an embodiment, the ridges may be positioned on the tunnel wall.

With such a configuration, the vision enhancer has a stronger frictional interaction with the adjacent tissue to the side of the vision enhancer.

In an embodiment, the ridges may alternatively or additionally be positioned on the tunnel base.

With such a configuration, the vision enhancer has a stronger frictional interaction with the adjacent tissue above the vision enhancer.

Depending on the geometry of the surgical tool, having ridges on the tunnel wall may result in difficulties upon demolding. In this event, the surgical tool could be produced by way of additive manufacturing.

In an embodiment, the ridges are not parallel to an axis of the vision enhancer.

With such a configuration, slippage in the direction of the axis of the vision enhancer in reduced, which is the most likely direction of slippage.

In an embodiment, one end of each ridge nearer a centreline of the tunnel base may be further from the tool body along the vision enhancer axis than an opposite end of the ridge.

With such a configuration, the ridges may act to part tissue adjacent the incision as the vision enhancer is passed through the incision.

In an embodiment, the tool may comprise a second end of the tool body, wherein an edge for separating tissue is situated at the second end.

With such a configuration, the same tool may be used for separating tissue to allow the vision enhancer to be inserted and for forming a tunnel in the tissue using the vision enhancer, reducing the overall number of tools needed.

In an embodiment, the edge for separating tissue may have a V-shape as viewed in a plan view, and/or a side view, perpendicular to the axis of the tool body, with the vertex of the V forming the end of the second end, preferably as viewed in two orthogonal directions, each being perpendicular to the axis of the tool body.

With such a configuration, the tissue may be separated and/or cut by way of an oscillating lateral movement of the tool, or an axial movement that is superimposed by rotation around an axis that is both, near the vertex of the V and approximately orthogonal to the plane of the V.

In an embodiment, the tool may comprise a guard situated proximal the second end of the tool.

With such a configuration, the surgeon is able to apply a force to the tool in a direction parallel to the longitudinal axis of the tool.

In an embodiment, the guard may have a face facing away from the second end of the tool, the face being at an angle between 75 degrees and 90 degrees, optionally between 75 degrees and 80 degrees, to the longitudinal axis of the tool body.

With such a configuration, the surgeon may apply a force with less chance of slippage against the face of the guard.

In an embodiment, the tool may further comprise an internal rib.

With such a configuration, shrinkage during an injection moulding process may be reduced.

A second object of the present invention is to provide a knife which has the dual purpose of being able to separate nerves from ligaments and cut the nerves without increasing the overall number of tools required.

According to a second aspect of the present invention, there is provided a surgical knife comprising: a tool body for gripping the tool having a longitudinal axis; a separating end; and a cutting end, wherein the separating end has a blunt tip for separating nerves from ligaments, and wherein the cutting end comprises: a blade for cutting a ligament; a first visual marker proximate the blade.

In an embodiment, the surgical knife may further comprise a discontinuous change in thickness of the tool, wherein the tool is thicker on the side of the discontinuous change further from the blade, the discontinuous change in thickness being situated further from the blade than the visual marker; and a gradual change in thickness of the tool extending from the discontinuous change in thickness away from the blade, the gradual change in thickness being such that the thickness of the tool increases with distance from the blade.

With such a configuration, the surgeon is made aware of the depth of insertion of the blade in order that insertion may be naturally slowed or more controlled during later parts of the incision, when the blade is more likely to encounter the ligament or nerves.

In an embodiment, the first visual marker may be situated 40 mm from a tip of the blade.

With such a configuration, the first visual marker is located a suitable distance from the tip of the blade for indicating approach of the transverse carpal ligament.

In an embodiment, the discontinuous change in thickness may be situated 45 mm from the tip of the blade.

With such a configuration, the step-change in thickness is appropriately located for indicating to the surgeon that the transverse carpal ligament is likely to have been reached.

In an embodiment, the gradual change in thickness may end at 52 mm from the tip of the blade.

With such a configuration, the gradual change in thickness is sufficiently long as to cover an expected depth of penetration without unduly affecting the length of the tool body.

In an embodiment, the separating end may have at least one, optionally two, second and/or third visual marker(s) situated proximate that blunt tip.

With such a configuration, the surgeon may be visually informed of the depth of insertion of the tool.

In an embodiment, the second and/or third visual marker(s) may be situated at 40 mm and/or 45 mm from the blunt tip.

With such a configuration, the second and/or third visual markers are appropriately positioned for indicating that the blunt tip is in the vicinity of the transverse carpal ligament

In an embodiment, the surgical knife may further comprise an internal rib.

With such an embodiment, shrinkage during an injection moulding production process may be reduced.

In a third object of the present invention, it is intended to provide a complete kit for performing surgery, having a cooperating pair of tools for preparing the area for surgery with a sufficient visual field and for performing the operation to separate the nerves and ligament and to cut the ligament.

According to a third aspect of the present invention, there is provided a surgical kit-of-parts comprising both the first and second aspects of the invention.

It is noted that, although the vision enhancer and edge for separating tissue are disclosed as being on the same tool, they may optionally be on separate tools and used and deployed independently.

Similarly, the surgical knife is disclosed as having a separating end and a cutting end, but may have only one of a cutting end or a separating end. In this way two separate, independent tools are envisaged, wherein each tool may have only one functional end.

### Brief Description of the Drawings

For a better understanding of the present invention, and to show how the same may be carried into effect, reference will now be made, by way of example only, to the following drawings, in which:
Figure 1 shows a general view of a surgical tool according to a first aspect of the present invention.
Figure 2 shows a side view of a surgical tool according to a first aspect of the present invention.
Figure 3 shows a cross section of a surgical tool according to a first aspect of the present invention, taken across line A-A of Figure 2.
Figure 4 shows a second end of a surgical tool according to a first aspect of the present invention.
Figure 5 shows a general view of a surgical knife according to a second aspect of the present invention.
Figure 6 shows a side view of a surgical knife according to a second aspect of the present invention.
Figure 7 shows a cutting end of a surgical knife according to a second aspect of the present invention.

### Detailed Description

The surgical tool 10 has a tool body 11, which may be held by a user when the surgical tool 10 is in use. A tool body axis 19 extends through the tool, substantially in line with the longest dimension of the tool. The surgical tool 10 also has a first end 12 and a second end 13.

The second end 13 of the surgical tool 10 has a separator edge 21. The separator edge extends from the tip of the surgical tool 10 toward the tool body 11. At one point the separator edge 21 splits to form a V-shape. The separator edge is formed so that it can cut tissue when moved to the side, perpendicularly to the tool body axis, in addition to, or alternatively to, a movement along the tool body axis 19.

In this way, the second end 13 and the separator edge 21 situated at the second end 13 may be used by a surgeon in order to separate tissue.

When using the surgical tool 10 to separate tissue, a surgeon may apply a force to the tool, in order to penetrate the tissue, along the tool body axis 19. When doing so, the surgeon may wish to rest a finger, thumb or side of the palm on a guard 22, and in particular on a face of the guard 23. The guard is therefore situated proximate the separator edge 21 and between the second end 13 and the tool body 11.

For this reason, the face of the guard 23 is at an angle of between 75 degrees and 90 degrees, preferably between 75 degrees and 80 degrees, to the tool body axis 19 so that the reaction force from the face of the guard 23 can directly oppose the force applied to the surgical tool 10 and the chance of slippage where the hand of the surgeon moves past the guard toward the second end 13 of the surgical tool 10 and the patient can be reduced.

The first end 12 of the surgical tool 10 has a vision enhancer 14. The purpose of the vision enhancer is to hold apart tissue, which has been separated using the separating edge 21 of the second end 13.

The vision enhancer 14 has a tunnel base 15, which extends from the tool body 11, and at least 1, preferably 2, tunnel wall(s) 16, which extend from the tunnel base 15. A groove 17 lies along a line joining the tunnel base 15 and tunnel wall(s) 16.

The tunnel base 15 and tunnel wall(s) 16 cooperate to form an internal concave surface. This concave surface then has a tunnel space within it. When inserted into a space under the skin, the tunnel wall(s) 16 and tunnel base 15 hold apart the tissue above the space and the tissue below the space so that an empty space is formed. This allows the surgeon to see more clearly the area upon which he or she is operating.

In order to be put into the surgery area, the vision enhancer must be inserted via an incision in the skin. When this is being done, the tunnel walls 16 may bend or hinge along the groove 17. When in the space under the skin, the tunnel walls 16 will then naturally move back to their position approximately perpendicular to the tunnel base 15 in order to give a sufficiently large tunnel and associated visual field for the surgeon to operate.

In order to provide the right compromise of flexibility and resilience in the hinging action, the tunnel wall thickness is between 0.75mm and 1.25mm, preferably 1 mm and the groove depth is preferably between 0.1 mm and 0.33 mm i.e. 10% to 30% of the tunnel wall thickness. The length of the groove can then be at least 30% of the length of the vision enhancer end, and up to the entire length of the vision enhancer end, optionally half the length of the vision enhancer end.

These dimensions are appropriate when the surgical tool 10 is made from a polymer, preferably polypropylene, more preferably a polymer comprising polypropylene having a melt flow index of 10 to 70cm/10 minutes, wherein the polypropylene is selected from polypropylene homopolymer or polypropylene copolymer having comonomer (2-8 carbon atoms) 1-10% by weight.

The tunnel base 15 has ridges 18. The ridges 18 provide an improved grip for reducing the chance of slippage during surgery. The ridges have a thickness at least 20% of the thickness of the tunnel base and/or tunnel wall, preferably between 20% and 70% of the thickness, more preferably 50%.

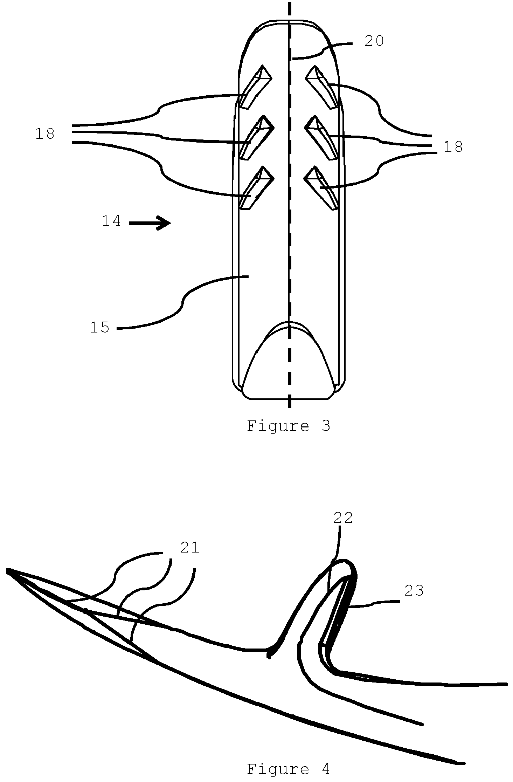

The ridges are angled to the vision enhancer axis 20 as shown in Figure 3. The angle is between 10° and 60°, preferably between 30° and 45°.

While the vision enhancer is shown has having a total of 6 ridges, the number of ridges may be increased or decreased.

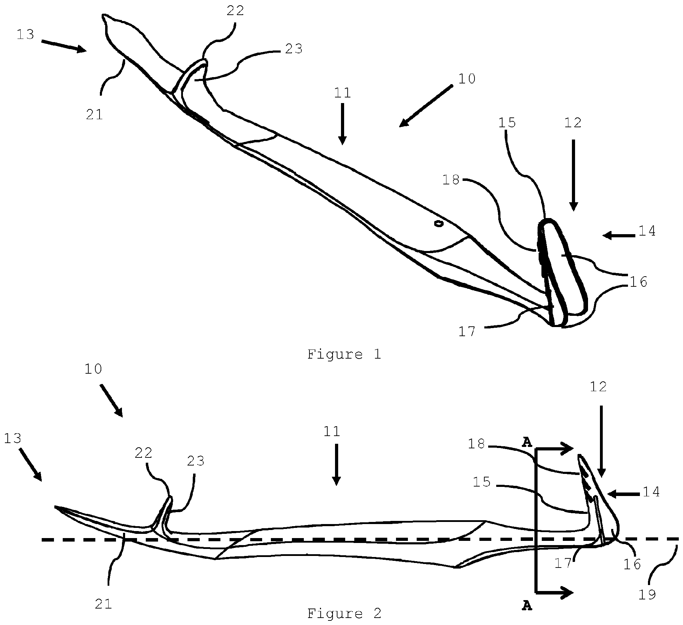

An end of the vision enhancer 14 situated furthest from the tool body 11 should be blunt for preventing injury to tissue, particularly during the insertion of the enhancer. Further, the tunnel wall(s) 16 may have an edge situated away from the tunnel base 15 which is designed for preventing tissue injury and for conforming to the wrist when the vision enhancer 14 is in use. Such an arrangement is shown in Figures 1 and 2.

Turning to the surgical knife 100, the surgical knife 100 has a tool body 101 which may be used by a surgeon to grip the surgical knife 100. The tool also has a separating end 102 and a cutting end 103.

The separator end 102 has a blunt tip 104, which can be used by the surgeon to separate the transverse carpal ligament from adjacent nerves in order to prevent nerves from being cut when the transverse carpal ligament is cut.

The width of the blunt tip is preferably between 4.5mm and 7.4mm, more preferably 5.9 mm, in order to allow easy insertion into the incision and the carpal tunnel, and optionally into the tunnel created by the vision enhancer 14.

A second and/or third visual marker 109a, b are situated proximal the blunt end, optionally 40 mm and/or 45 mm from the blunt tip 104. This gives a visual indication to the surgeon when the blunt tip is in the vicinity of the transverse carpal ligament.

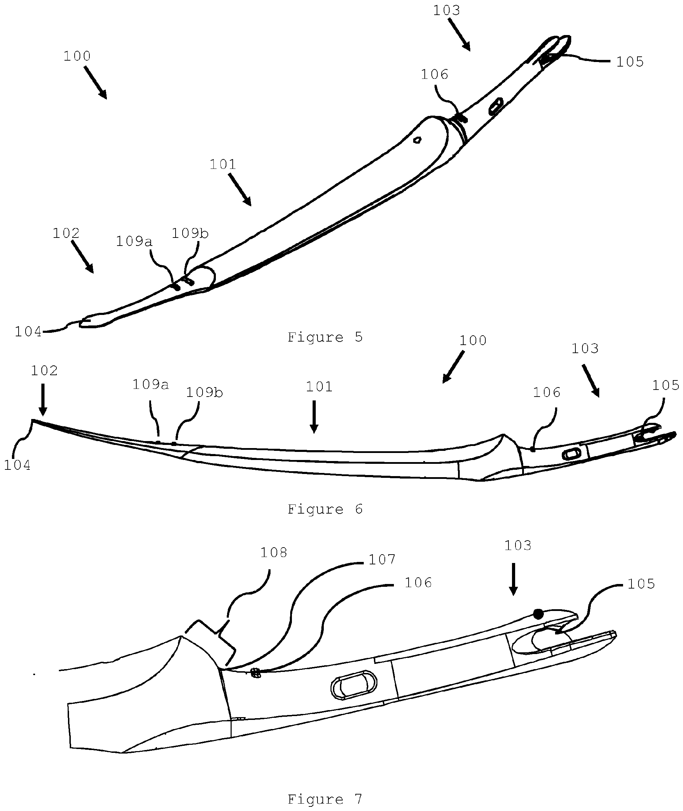

The cutting end 103 has a blade 105 for cutting of the transverse carpal ligament. The blade has an upper and lower cutting end as shown in Figures 5, 6 and 7. The thickness of the lower cutting end is between 1.875mm and 3.125mm, preferably 2.5 mm and the thickness of the upper cutting end is between 1.95mm and 3.25mm, preferably 2.6 mm. Thin cutting sections make visualisation of the surgical area easier due to the transparency of the material.

The surgical knife 100 has a first visual marker 106 situated proximal the cutting end 103, between 37mm and 43mm from the tip of the blade 105, preferably between 38mm and 41mm from the tip of the blade 105 more preferably between 39mm and 40mm from the tip of the blade 105; in a most preferred embodiment the visual marker 106 is situated about 40 mm from the tip of the blade 105. This allows the surgeon to visually tell when the tip of the blade is in the vicinity of the transverse carpal ligament, since the transverse carpal ligament is usually approximately 40 mm from the incision.

The surgical knife 100 initially maintains its thickness, then gradually increases in thickness with increasing distance from the tip, before reaching a discontinuity in the change of thickness. This discontinuity can also be considered as a discontinuous change in thickness 107, where the surgical knife 100 is thicker on the side of the discontinuous change further form the cutting end 103. The tool body then again has a gradual change in thickness 108 with the tool body becoming thicker as the distance from cutting end 103 increases, as shown in figure 7.

The discontinuous change and gradual change in thickness 107, 108 have the effect of giving a physical notification to the surgeon that the depth of insertion of the surgical knife 100 is beyond that which is usually required for cutting of the transverse carpal ligament. For this reason, the step change is situated between 43mm and 48mm from the tip of the blade 105, preferably between 44mm and 47 mm from the tip of the blade 105, more preferably between 45mm and 46mm from the tip of the blade 105; in a most preferred embodiment the step change is situated 45 mm from the tip of the blade 105 and the gradual change in thickness 108 extends to a point between 48mm and 53mm from the tip of the blade 105, preferably between 50mm and 53mm from the tip of the blade 105, more preferably between 51mm and 52mm from the tip of the blade 105; in a most preferred embodiment, the gradual change in thickness 108 extends to a point 52 mm from the tip of the blade 105.

These distances are selected based on collected data, which found that the width of the transverse carpal ligament is between 38mm and 39mm in 90 to 95% of cases.

The surgical tool 10 and the surgical knife 100 may be formed by an injection moulding process. In this process the surgical tool 10 and surgical knife 100 may each be formed in two separate parts and inserted together subsequently.

For example, the surgical tool 10 or surgical knife 100 may be manufactured partially from a hard polymer and partially from a soft rubber in order to make the tool easier to grip, while maintaining adequate structural stiffness. It is noted that other material combinations are available, but that the two-part manufacture allows a greater range of properties to be obtainable for the tool 10 or knife 100.

During such a process, the products of the process are liable to suffer from shrinkage. This can be reduced by the surgical knife 10 or surgical tool 100 comprising an internal rib.

The rib may be formed such that one part of the product has an internal rib and the second part has a corresponding recess, such that the internal rib of the first part is inserted into the corresponding recess of the second part.

The surgical tool 10 and surgical knife 100 cooperate such that in use the surgical tool 10 may be used to separate tissue and then to form a tunnel, with the surgical knife 100 being inserted via said tunnel in order to separate the nerves and ligament and to cut the ligament.

Apart from the two aforementioned exemplary embodiments, which teach the manner in which the invention may be carried into effect, further alternative embodiments are possible. In particular, there are a number of variations which are possible, as may be appreciated by those skilled in the art.

For example, the vision enhancer 14 may not comprise a groove; the vision enhancer 14 may have a material which is weakened along a line between a tunnel wall 16 and the tunnel base 15. This would facilitate the bending of the vision enhancer as required.

In another embodiment, the ridges 18 on the vision enhancer 14 may not be present. A frictional coating may be provided by a rubberised coating on the tunnel base 15.

In a further exemplary embodiment, the vision enhancer may have an asymmetric arrangement, with only a single tunnel wall being present.

The surgical tool 10 may have no guard 22, or a guard with no face 23 perpendicular to the tool body axis 19. In this case, the tool might have ridges or a soft rubber coating in order to facilitate sufficiently strong gripping without risk of slipping of the surgeons hand during the procedure.

It is noted that all of the positions of visual markers and/or physical markers are exemplary and have been chosen by the inventors as the anatomical match of the average carpal tunnel syndrome patient population. Differing positions of such markers are possible, such as for surgery performed on children or smaller adults.

All of the above are fully within the scope of the present invention, and are considered to form the basis for alternative embodiments in which one or more combinations of the above-described features are applied, without limitation to the specific combinations disclosed above.

In light of this, there will be many alternatives which implement the teaching of the present invention. It is expected that one skilled in the art will be able to modify and adapt the above disclosure to suite its own circumstances and requirements within the scope of the present invention, while retaining some or all technical effects of the same, either disclosed or derivable from the above, in light of his comment general knowledge in this art. All such equivalents, modifications or adaptations fall within the scope of the invention hereby defined and claimed.

### The following items are also part of the invention

1. A surgical tool comprising:
   a tool body for gripping the tool, the tool body having
   a longitudinal axis and a first end,
   wherein a vision enhancer extends from the tool body at the first end, the vision enhancer comprising:
   a tunnel base, and
   a first tunnel wall extending from the tunnel base at an angle of between 90 and 120 degrees to the tunnel base,
   wherein the tunnel base and the first tunnel wall form a concave section for the forming of a tunnel through soft tissue.
2. The surgical tool of item 1, wherein the vision enhancer has a groove extending along a line joining the first tunnel wall and the tunnel base.
3. The surgical tool of item 1 or 2, further comprising a second tunnel wall extending from the tunnel base at an angle of between 90 and 120 degrees to the tunnel base and parallel to the longitudinal axis of the tool body, the second tunnel wall preferably being spaced from the first wall, in a direction perpendicular to the longitudinal axis, by the dimension of the tunnel base in that direction.
4. The surgical tool of item 3, wherein the vision enhancer has at least two grooves, each groove extending along a line joining one of said tunnel walls and the tunnel base.
5. The surgical tool of any preceding item, wherein the concave section formed by the tunnel base and the first tunnel wall defines an inside of the vision enhancer, and wherein the vision enhancer further comprises ridges on the outside of the vision enhancer.
6. The surgical tool of item 5, wherein one end of each ridge nearer a centreline of the tunnel base is further from the tool body along the vision enhancer axis than an opposite end of the ridge.
7. The surgical tool of any preceding item, wherein the tool body further comprises a second end, an edge for separating tissue being situated at the second end.
8. The surgical tool of item 7, wherein the edge for separating tissue has a V-shape, in plan view, and/or side view, as viewed perpendicularly to the longitudinal axis of the tool body.
9. The surgical tool of item 7 or 8, further comprising a guard situated proximal the second end of the tool.
10. The surgical tool of item 9, wherein the guard has a face facing away from the second end, the face being at an angle of between 75 degrees and 90 degrees, optionally between 75 degrees and 80 degrees, to the longitudinal axis of the tool body.
11. A surgical knife comprising:
   a tool body for gripping the tool having a longitudinal axis;
   a separating end; and
   a cutting end,
   wherein the separating end has a blunt tip for separating nerves from ligaments, and
   wherein the cutting end comprises:
   a blade for cutting a ligament;
   a first visual marker proximate the blade.
12. The surgical knife of item 11, further comprising a discontinuous change in thickness of the tool, wherein the tool is thicker on the side of the discontinuity further from the blade, the discontinuous change in thickness being situated further from the blade than the visual marker; and
   a gradual change in thickness of the tool extending from the discontinuous change in thickness away from the blade, the gradual change in thickness being such that the thickness of the tool increases with distance from the blade.
13. The surgical knife of item 11 or 12, wherein the separating end has at least one, optionally two, second and/or third visual marker(s) situated proximate the blunt tip.
14. The surgical knife of items 11, 12 or 13 or the surgical tool of any one of items 1 to 15, further comprising an internal rib.
15. A surgical kit-of-parts comprising the surgical tool of any one of item 1 to 10 and the surgical knife of any one of item 11 to 14.

## Claims

1. A surgical knife comprising:
a tool body for gripping the tool having a longitudinal axis;
a separating end; and
a cutting end,
wherein the separating end has a blunt tip for separating nerves from ligaments, and
wherein the cutting end comprises:
a blade for cutting a ligament;
a first visual marker proximate the blade.

2. The surgical knife of claim 1, further comprising a discontinuous change in thickness of the tool, wherein the tool is thicker on the side of the discontinuity further from the blade, the discontinuous change in thickness being situated further from the blade than the visual marker.

3. The surgical knife of claim 2, further comprising a gradual change in thickness of the tool extending from the discontinuous change in thickness away from the blade, the gradual change in thickness being such that the thickness of the tool increases with distance from the blade.

4. The surgical knife of any preceding claim, wherein the separating end has at least one, optionally two, second and/or third visual marker(s) situated proximate the blunt tip.

5. The surgical knife of any preceding claim, further comprising an internal rib.

6. The surgical knife of any preceding claim, wherein the surgical knife is formed by an injection moulding process, and wherein preferably at least a part of the surgical knife is made from a transparent material.

7. The surgical knife of any of claims 5 or 6, wherein the internal rib is configured to reduce shrinkage during an injection moulding process.

8. The surgical knife of any preceding claim, wherein the internal rib is provided on a first part of the surgical knife, and wherein the internal rib is configured to be inserted into a corresponding recess of a second part of the surgical knife.

9. The surgical knife of any preceding claim, wherein the visual marker is situated between 37 mm and 43 mm from the tip of the blade, preferably between 38 mm and 41 mm from the tip of the blade, more preferably between 39 mm and 40 mm from the tip of the blade, and most preferably 40 mm from the tip of the blade.

10. The surgical knife of any preceding claim, wherein the visual marker is disposed so as to allow a medical practitioner to visually tell when the tip of the blade is in a vicinity of an element to be cut by the surgical knife.

11. A surgical kit-of-parts comprising the surgical knife of any preceding claim and a surgical tool, wherein the surgical tool comprises:
a tool body for gripping the tool, the tool body having a longitudinal axis and a first end,
wherein a vision enhancer extends from the tool body at the first end, the vision enhancer comprising:
a tunnel base, and
a first tunnel wall extending from the tunnel base at an angle of between 90 and 120 degrees to the tunnel base,
wherein the tunnel base and the first tunnel wall form a concave section for the forming of a tunnel through soft tissue.

12. A method for providing a surgical knife according to any of claims 1-10, wherein the method comprises:
- providing a first part of a surgical knife having a rib; and
- inserting the rib of the first part into a corresponding recess of a second part of the surgical knife.

13. The method for providing a surgical knife according to claim 12, wherein the method further comprises an injection moulding process.
